# EUROPEAN PATENT APPLICATION

(11) **EP 4 311 489 A1**
(43) Date of publication of application: **31.01.2024**
(21) Application number: 22187986.9
(22) Date of filing: 30.07.2022
(51) Int. Cl.: A61B 5/117, A61B 5/00, A61B 5/332, A61B 5/346

(54) **METHOD AND DEVICE FOR USING ECG SIGNALS FOR PERSONAL AUTHENTICATION**

(71) Applicant: Cardioid a.s., 60200 Brno (CZ)
(72) Inventor: Repcík, Tomás, 05801 Poprad (SK); Matausch, Robertino, 85777 Fahrenzhausen (DE)

(57) **Abstract**

Method and device for using ECG signals for personal authentication by converting raw ECG signals into individual and unambiguous ECG signal periods, which are divided into individual sequences by heartbeat detection. These series of ECG signals are individual, unique and suitable for one-time registration of the neural network artificial intelligence algorithm, which can be used by an authentication device, either stand-alone, such as in the form of a token, or embedded in some form of chipset or hardware.

## Description

Method and Apparatus for using ECG signals for personal authentication by converting raw ECG signals into individual and unambiguous ECG signal periods, which are divided into individual sequences by heartbeat detection.

### INTRODUCTION:

ECG sensors are widely available at low cost and have been integrated in e.g. smart watches. However, biometric identification and authentication has been lagging primarily for lack of consistent and reliable pattern recognition systems.

Therefore, the static features of ECG cannot be used for personal identification without further processing, since ECG signals are essentially temporal (i.e. time-varying) patterns with significant variations ("noise"). Thus, the identity recognition based on ECG signals is actually a temporal pattern recognition problem that is solved by presented invention.

It should be noted that the technology in most pulse monitors does not record ECG. Heart monitor fitness-trackers generally use optical sensors to measure blood-flow and not electrical activity. In rare cases this can even be observed over a distance and is therefore completely unsuitable for biometric systems.

Unlike the present invention, the comparable ECG biometrics using deep learning with the success rate of identifying an individual within a closed group. Contrary to that, the present invention is based on the verification principle.

Existing verification-oriented works that are based on fiducial points, ECG morphology, and other methods. These works (not based on deep-learning) achieve high equal error rate (EER or comparable error rate, where the FAR and FRR are equal), in particular: Extraction of morphological and interval features Application of local binary patterns to individual heartbeats (ERR EER: 12,63 %), Application of local binary patterns to individual heartbeats (FAR: 1,57 FRR: 0,39), Averaging and filtering individual heartbeats and comparison using different distance metrics (FAR: 5,8 FRR: 11,6), Combining linear discriminant analysis and multi-view learning (ERR: 2,68 ERR: 0,34 ERR: 1,97 ERR: 0,03), Fiducial point search and analysis using machine learning methods (FAR: 5,71 FRR: 3,44), Template formation based on dimension reduction and identity search using multiple signal classification algorithm (using multiple leads) (ERR: 0,038), Creating a sparse representation of a heartbeat using regularization (ERR: 0,58).

The main advantage of these methods is the possibility of applying the algorithm even to a few heartbeats. This provides computational efficiency and possibility to apply algorithms for datasets, which contain a small number of measurements per included person.

However, in terms of success rates, most algorithms achieve higher average error rates than present invention, even for smaller database sizes.

### THE BACKGROUND ART:

A biometric system performs template-matching of acquired biometric data against template biometric data. These biometric data can be acquired from several sources, including deoxyribonucleic acid (DNA), face, facial thermogram, fingerprints, gait, hand geometry, hand veins, iris, palm print, retina, voice, etc. According to previous research, DNA-derived characteristics provide high measurement accuracy for biometric identifiers including universalities, distinctiveness and performance.

DNA provides a one-dimensional ultimate unique code for accurate identification for a person, except for the case of identical twins.

The composition, mechanism and electrical activity of the human heart inherit their uniqueness from the individuality of DNA. An electrocardiogram (ECG) represents the electrical activities of the heart.

Conventional biometric security technology, while broadly available, suffers from specific drawbacks: (i) often, they require highly sensitive and expensive lens systems (iris, facial recognition), (ii) impeding factors such as lighting or ambient conditions lead to frequent errors or rejections, and (iii) the biometric "template", i.e. face, iris or fingerprint are often easily duplicated (photographs, "lifted" fingerprints) and may then be used to breach security via identity theft. While more precise systems are available to detect counterfeit templates, such systems are more expensive and vulnerable to more sophisticated forgeries. Conversely, an electrocardiogram (ECG) is the recording of the heart's electrical activity and can be easily measured from the body surface. Its nature is uniquely personalized, since it relies on the properties of heart structure and function; thus, it can be used as a biometric trait for identity recognition. Compared to other biometric traits, ECG has significant advantages for identity recognition: (i) it is near impossible to forge and can only be measured from living individuals; (ii) it has a good stability and reproducibility; (iii) it contains information pertaining to psychological, physiological and clinical states, which may be of ancillary interest. Extensive studies have confirmed ECG as a unique biometric identification characteristic over the last fifteen years.

The invention uses the adaptable algorithms and artificial intelligence. The invention tracks the electrical stimulations inside the heart that power the mechanical activity of the human heart (depolarization (electrical activity) of a specific portion of the heart (either atria or ventricle) results in mechanical contraction of that specific part, repolarization results in the mechanical relaxation of the heart chambers).

Previous research has classified the ECG based biometric techniques in two ways. The first methodology is an ECG biometric measured in millivolt with Fiducial Point detection (i.e. "marker points" that collectively define the shape). Under this process, on-set and off-set of the feature waves are detected first. After locating all these points, feature wave duration, amplitude, curvature, direction, slope, etc., are obtained. These wave characteristics are saved as enrollment data. During the recognition phase, all this information is again extracted from the recognition ECG. At last, template matching of the ECG morphology is performed.

In the second type of ECG biometric, ECG features are extracted in a frequency domain. Therefore, the ECG signal as measured in millivolts is converted to the frequency domain using finite sampling intervals, in which the desired ECG features are identified. These frequency domain transformations may utilize various signal processing techniques like Fourier Transform, Wavelet Transform, Discrete Cosine Transform, etc.

Both of these conventional methodologies have significant drawbacks that adversely affect the reliability and the precision with which ECGs can be identified, especially under conditions in which the heartbeat and consequently the ECG deviate from the ideal basic pattern in calm conditions, e.g. after strenuous activity, excitement or impaired health conditions.

### INVENTION DISCLOSURE:

Unlike Fiducial Point and Frequency methods, the invention uses the Convolutional Neural Network (CNN) processing. CNNs are deep learning algorithms which can take in an input image or data string, assign importance (learnable weights and biases) to various aspects/objects in the image or data convolution and be able to differentiate one from the other. The pre-processing required in a CNN is much lower as compared to other classification algorithms. While, in primitive methods, filters are hand-engineered, with enough training, CNN have the ability to learn these filters/characteristics by themselves. While "healthy" ECG signals from different individuals correspond to nearly the same repetitive pulse pattern, small differences throughout the waveform as a whole enable significant differentiation between individual people. During the authentication process, the invention is able to ignore the "noise" caused by breathing, movement or unstable connections, and will focus entirely on pattern recognition to accept or reject the user (1). The pattern recognition software uses a combination of downstream statistical methods and deep machine learning methods to extract the unique ECG features and simultaneously reject unwanted artifacts - errors, noise and random patterns. During the enrollment, the software generates a unique model of an individual's ECG that is clearly distinguishable within a random population.

The invention uses the base model within the CNN that eliminates the "distorted" ECGs in deviating conditions of the heart, and leads to high verification accuracy. Thus, the individual heartbeats are traced. For this purpose any kind of R wave detector can be used (2).

The quality of the ECG signal needs to be evaluated to prohibit evaluation of random signals inputted into biometry algorithm. Consequently after the R wave detection, the heartbeats are cropped out of the signals (2). The series of heartbeats needs to be normalized in range and length, but techniques like filtration, segmentation, aggregation are applied. Modified series of heartbeats are analyzed by deep learning framework engineered for every individual person (3).

The deep learning framework is composed of three parts. First part is focused on extracting unique features, with which help the person can be verified. The extraction can be handled by various types of the convolution neural networks or other state of the art techniques in feature extraction (4). The second part contains mechanism, which uses extracted features and transforms them into representative encoded form. The representation contains all the required information to verify the person. Representation can be derived by trained feed-forward neural networks or other architectures. Third part, decision making part, takes representation of the person and compares it with template (5). As security improvement, the template could be replaced with quantified metric, so no reference to actual person can be inferred. With use of statistical significance of similarities, the algorithm can authenticate or deny the analyzed person (6).

In order to enroll new users, they are required to measure their ECG for extended time period to acquire representative data of each of them. The dynamic creation of deep learning framework takes place at cloud, which puts ECG data of our person against database of other people.

The deep learning technique exploits uniqueness of other people to find unique features of every person. To achieve the highest accuracy, learning phase is lead by optimization and training algorithm, which can optimize specialized architecture of neural networks embedded in framework. In the end, every deep learning framework is personalized for the unique characteristics of the person and it is self-sufficient to verify the person's identity and deny all other people.

### CARRYING OUT THE INVENTION CLAIMED:

The invention may be used in appliances that share the same principles: the device that requires personal authentication will carry a sensor chip that measures the actual ECG.

The sensor chip is based on a module that includes an ECG sensor with two groups of electrodes (one on the inside and one on the outside of the device). Similar to an Activity Tracker, one electrode touches the wrist or similar body part, while the other one is exposed towards the back. An ECG is detected when the user touches the electrode at the top with the opposite hand.

The Identification Process: For its use, the device that uses the invention requires a so-called Authorized Authentication Device (AAD). An AAD can be a logical part of the device or a smartphone, tablet or computer, but also a badge reader, a cash machine or any other device that communicates via Bluetooth with the device. The AADs enables the user both the so-called enrollment as well as the authenticate process.

Enrollment: An enrollment is the process when a master ECG is recorded and processed in order to use it as a biometric master ID template. It is important to note that this master ID template is actually a CNN-generated template, not the ECG data itself. The enrollment process is initiated and executed from an AAD. The user's ECG is recorded by the device and transmitted wirelessly to the AAD via a secure encrypted connection. The biometric master template is encrypted and stored on the AAD and cannot be compromised even if the AAD itself has been compromised. Depending on configuration, the AAD can provide instances of the biometric master template to individual devices or for central storage on an authentication server accessed by such devices.

Authentication: Authentication is the process when the identity of the user is matched against the previously created biometric master ID template. As with the enrollment, the user's ECG is recorded with the device and transmitted to instance holding the biometric master template (e.g. the authentication server or the AAD itself). The recorded live ECG is compared in real time with the biometric master template. If the matching occurs within the maximum amount of time permitted, the user is authenticated and the device is activated. As soon as the device is in a so-called authenticated state, it will be able to communicate with other enabled devices and/or services. If this initialization process is completed, the AAD is no longer required.

The invention makes sure that the medical heart conditions such as cardiac arrhythmia, arterial flutter, or implants (e.g. pacemaker) do not affect the operation of the device. The reason for this is that every heartbeat, even an abnormal one, has its own unique signature, and precisely because these intrinsic patterns are permanent, the device based on present invention can evaluate, analyze and store them in the biometric master template within a learning process.

The apparatus is able to consider and eliminate the low frequency anomalies that cause the slight variations in the heart rhythm resulting from, for example, through activities such as light exercise, caffeine use, medication, or meditation.

### INDUSTRIAL APPLIANCE:

The use of secure authentications opens number of scenarios, such as: (i) current distributed digital identity schemes based on blockchain, (ii) Smart Contracts with proven identity, (iii) Defense applications such as access to weapons and weapon systems, premises control, military data protection, command communication, verification of authorization and information access levels, protection of networks and devices, (iv) Healthcare Management The invention may be used in following scenarios: (a) Continuous Use Option including liveliness detection: The authentication is performed only once when the user picks up the device (e.g., early morning). Based on continuous proximity of the device to the authenticated user, authorized processes and systems can continue to be used. After authentication, the device (e.g. a smartwatch) remains part of the user who can then pursue all his usual activities. During this time, access to all connected devices will be authorized until the device is taken off or removed from immediate proximity. Once the device is removed from proximity, any access to such device will require renewed authentication, (b) Three-Factor-Authentication that uses 3 co-dependent steps: (i) Biometric Authentication/ Verification based on ECG, (ii) Physical Possession of the authentication device, and (iii) Physical Possession of the Authorized Authentication Device that is used for initial authentication (c) Public identification and secure digital signing of transactions: Besides using in authentication devices, the invention may be used for public identification and secure digital signing of transactions. By using the Ecliptic Curve Digital Signature Algorithm (ECDSA) integrated into the hardware the secure key pairs (public and private) are generated. This circumvents the use of "security token" that communicates with remote server by using a public key encryption, which means that there is only one self-destructible copy of the private key in the device (4) Random Key Generation and Storage: Block ciphers such as AES are often used in popular applications. The device provides a secure and convenient way to manage cryptographic keys used by those applications. To enable the correct use and storage of block cipher keys, the device provides a hardware-based, random number generator that allows a multitude of applications to generate random numbers and retrieve them when required. This allows the owner of the device to use it as a safe set of keys and to wear it permanently.

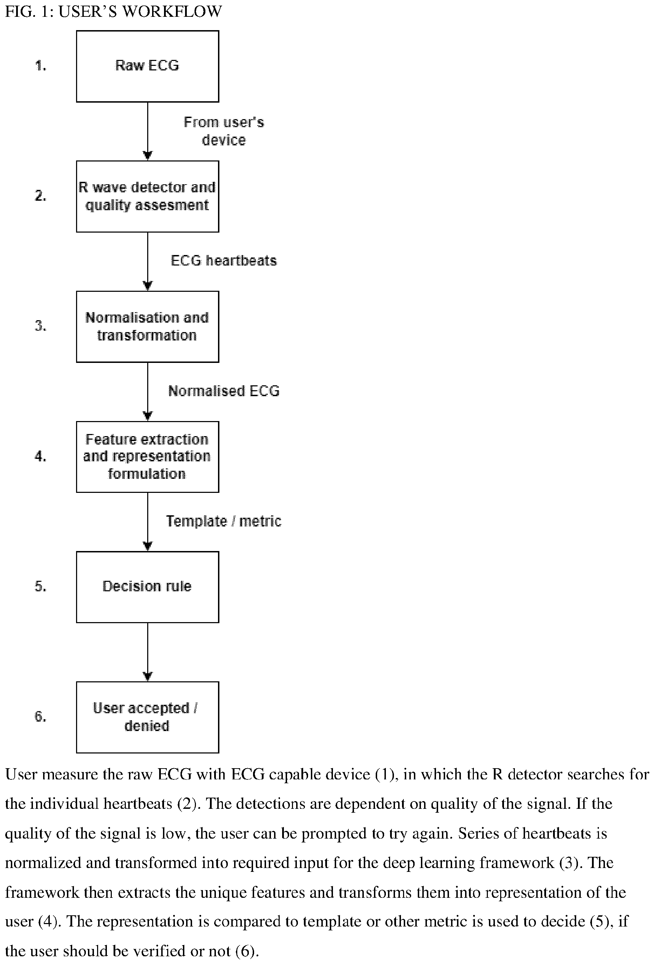

## Claims

1. A method of converting raw ECG (electrocardiogram) signals into individual and unambiguous ECG signal periods, which are divided into individual sequences by heartbeat detection, suitable for use of ECG signals for authentication of individual person, **characterized by**:
one-time initial processing the master raw ECG data taken from individual person into normalized ECG signal periods attributable to certain individual person by virtue of distinctive features that are clear of noise signals, and that is unambiguous enough to guarantee the sufficient probability of un-doubtful authentication of a person, and that is simple enough to be processed by personal device or locally;
one-time enrollment of the neural network artificial intelligence algorithm-generated individual heartbeat sequences pattern into the individual authentication device, either stand-alone, such as in the form of a token, or embedded in some form of chipset or hardware; receiving ECG signals measured by personal device anytime when the authentication is performed;
wherein said device measures the raw ECG of individual person (1), processes it (2) into the normalized ECG (3), simplifies it to the heartbeats pattern template (4), and verifies it against the existing legit individual metric in order to authenticate the individual person (5) 2. An electronic device that contains a chipset in accordance with claim 1. wherein the chipset measures raw ECQ from the person, and compares it to enrolled user profile that consists of neural-network generated individual heartbeats pattern.
